# EUROPEAN PATENT APPLICATION

(11) **EP 4 666 872 A1**
(43) Date of publication of application: **24.12.2025**
(21) Application number: 24756888.4
(22) Date of filing: 13.02.2024
(51) Int. Cl.: A23L 33/135, A23C 9/13, A23G 9/36, A61K 35/747, A61P 37/04, C12N 1/20

(54) **LACTIC ACID BACTERIA AND IMMUNOSTIMULANT**

(30) Priority: 13.02.2023 JP 2023020334
(71) Applicant: Ezaki Glico Co., Ltd., Osaka-shi Osaka 555-8502 (JP)
(72) Inventor: TSURUNO, Keigo, Osaka-shi, Osaka 555-8502 (JP); MAWATARI, Takashi, Osaka-shi, Osaka 555-8502 (JP)
(74) Representative: Hoffmann Eitle
(86) International application number: PCT/JP2024/004894
(87) International publication number: WO 2024/172046

(57) **Abstract**

The present invention provides an agent for immune stimulation containing *Lactobacillus helveticus,* wherein the *Lactobacillus helveticus* has three functions: dendritic cell-like cell activation, IgA production induction, and IL-12 production induction.

## Description

### Technical Field

The present invention relates to lactic acid bacteria of the genus *Lactobacillus* and agents for immune stimulation.

### Background Art

The immune system plays a crucial role in maintaining and improving health, and research on the immunity has been conducted in various fields. There are two types of immunity: innate immunity and acquired immunity, which are both responsible for the body's defense functionality. Due to increasing health consciousness, there is strong demand for means to enhance immune functions. Consuming lactic acid bacteria is known to enhance such immune functions. There are various types of lactic acid bacteria, and lactic acid bacteria that belong to the genus *Lactobacillus* are industrially useful due to their application in a wide variety of foods, such as in the production of yogurt. Therefore, there is strong demand for the development of lactic acid bacteria of the genus *Lactobacillus* with high immunostimulatory activity.

### Citation List

### Patent Literature

PTL 1: Japanese Patent No. 6170190
PTL 2: Japanese Patent No. 6652331
PTL 3: Japanese Patent No. 6705628
PTL 4: Japanese Patent No. 6796299
PTL 5: Japanese Patent No. 5968655

### Summary of Invention

### Technical Problem

An object of the present invention is to provide a novel agent for immune stimulation with high immunostimulatory activity using lactic acid bacteria of the genus *Lactobacillus.* In a preferred embodiment, an object of the present invention is to provide novel *Lactobacillus* species that exhibit higher immunostimulatory activity than the known lactic acid bacteria strain JCM 5805, and an agent for immune stimulation using the *Lactobacillus* species.

### Solution to Problem

In such circumstances, the present inventors conducted research on various lactic acid bacteria from the viewpoint of immune stimulation, and discovered multiple lactic acid bacterial species with high immunostimulatory activity from among countless existing lactic acid bacteria. Moreover, the inventors discovered that among *Lactobacillus helveticus* species, which belongs to the genus *Lactobacillus,* certain strains exhibit high activity. The present invention is based on this novel finding. Thus, the present invention provides an agent for immune stimulation, lactic acid bacteria, and other subject matter shown in the following Items:
Item 1.
   An agent for immune stimulation, comprising *Lactobacillus helveticus* as an active ingredient, wherein the *Lactobacillus helveticus* has three functions: dendritic cell-like cell activation, IgA production induction, and IL-12 production induction.
Item 2.
   The agent for immune stimulation according to Item 1, wherein the *Lactobacillus helveticus* is *Lactobacillus helveticus* GCL1815 or *Lactobacillus helveticus* GCJ5-4B.
Item 3.
   The agent for immune stimulation according to Item 1 or 2, wherein the *Lactobacillus helveticus* satisfies at least one requirement selected from the group consisting of the following (1) to (3):
   (1) when the *Lactobacillus helveticus* is added at 1 × 10⁷ cells/well to a well seeded with 250 µL of human-derived peripheral-blood mononuclear cell fluid at a concentration of 8 × 10⁵ cells/mL, 100 ng/mL or more of IgA is produced;
   (2) when the *Lactobacillus helveticus* is added at 4 × 10⁶ cells/well to a well seeded with 200 µL of human-derived dendritic cell-like cells at a concentration of 1.25 × 10⁵ cells/mL, CD86 expression intensity becomes at least 1.5 times higher than that of a control without any lactic acid bacteria; and
   (3) when the *Lactobacillus helveticus* is added at 4 × 10⁶ cells/well to a well seeded with 200 µL of human-derived dendritic cell-like cells at a concentration of 1.25 × 10⁵ cells/mL, 300 pg/mL or more of IL-12 is produced.
Item 4.
   The agent for immune stimulation according to Item 3, wherein the *Lactobacillus helveticus* satisfies the three requirements (1) to (3).
Item 5.
   The agent for immune stimulation according to any one of Items 1 to 4, wherein at least one immunostimulatory function among the three immunostimulatory functions, the dendritic cell-like cell activation, the IgA production induction, and the IL-12 production induction possessed by the *Lactobacillus helveticus,* is higher than that of *Lactococcus lactis* JCM 5805 strain.
Item 6.
   The agent for immune stimulation according to any one of Items 1 to 5, wherein the three immunostimulatory functions, the dendritic cell-like cell activation, the IgA production induction, and the IL-12 production induction, possessed by the *Lactobacillus helveticus,* are higher than those of *Lactococcus lactis* JCM 5805 strain.
Item 7.
   The agent for immune stimulation according to any one of Items 1 to 6, wherein the immune stimulation is maintenance of immune function in a healthy individual.
Item 8.
   An oral composition for immune stimulation, comprising the agent for immune stimulation of any one of Items 1 to 7.
Item 9.
   The oral composition according to Item 8, which is a composition for food or drink.
Item 10.
   A prophylactic or therapeutic agent for viral infection, comprising the agent for immune stimulation of any one of Items 1 to 7.
Item 11.
   A lactic acid bacterium that belongs to the genus *Lactobacillus,* with accession number NITE BP-03804 at the NITE Patent Microorganism Depository (NPMD) of the National Institute of Technology and Evaluation (NITE).
Item 12.
   A lactic acid bacterium that belongs to the genus *Lactobacillus,* with accession number NITE BP-03805 at the NITE Patent Microorganism Depository (NPMD) of the National Institute of Technology and Evaluation (NITE).
Item 13.
   A lactic acid bacterium for use in immune stimulation, wherein the lactic acid bacterium is *Lactobacillus helveticus* that possesses three functions: dendritic cell-like cell activation, IgA production induction, and IL-12 production induction.
Item 14.
   The lactic acid bacterium for use according to Item 13, wherein the lactic acid bacterium is *Lactobacillus helveticus* GCL1815 or *Lactobacillus helveticus* GCJ5-4B.
Item 15.
   The lactic acid bacterium for use according to Item 13 or 14, wherein the *Lactobacillus helveticus* satisfies at least one requirement selected from the group consisting of the following (1) to (3):
   (1) when the *Lactobacillus helveticus* is added at 1 × 10⁷ cells/well to a well seeded with 250 µL of human-derived peripheral-blood mononuclear cell fluid at a concentration of 8 × 10⁵ cells/mL, 100 ng/mL or more of IgA is produced;
   (2) when the *Lactobacillus helveticus* is added at 4 × 10⁶ cells/well to a well seeded with 200 µL of human-derived dendritic cell-like cells at a concentration of 1.25 × 10⁵ cells/mL, CD86 expression intensity becomes at least 1.5 times higher than that of a control without any lactic acid bacteria; and
   (3) when the *Lactobacillus helveticus* is added at 4 × 10⁶ cells/well to a well seeded with 200 µL of human-derived dendritic cell-like cells at a concentration of 1.25 × 10⁵ cells/mL, 300 pg/mL or more of IL-12 is produced.
Item 16.
   The lactic acid bacterium for use according to Item 15, wherein the *Lactobacillus helveticus* satisfies the three requirements (1) to (3).
Item 17.
   The lactic acid bacterium for use according to any one of Items 13 to 16, wherein at least one immunostimulatory function among the three immunostimulatory functions, the dendritic cell-like cell activation, the IgA production induction, and the IL-12 production induction possessed by the *Lactobacillus helveticus,* is higher than that of *Lactococcus lactis* JCM 5805 strain.
Item 18.
   The lactic acid bacterium for use according to any one of Items 13 to 17, wherein the three immunostimulatory functions, the dendritic cell-like cell activation, the IgA production induction, and the IL-12 production induction, possessed by the *Lactobacillus helveticus,* are higher than those of *Lactococcus lactis* JCM 5805 strain.
Item 19.
   The lactic acid bacterium for use according to any one of Items 13 to 18, wherein the immune stimulation is maintenance of immune function in a healthy individual.
Item 20.
   A composition for immune stimulation, comprising *Lactobacillus helveticus* as an active ingredient, wherein the *Lactobacillus helveticus* has three functions: dendritic cell-like cell activation, IgA production induction, and IL-12 production induction.
Item 21.
   The composition according to Item 20, wherein the *Lactobacillus helveticus* is *Lactobacillus helveticus* GCL1815 or *Lactobacillus helveticus* GCJ5-4B.
Item 22.
   The composition according to Item 20 or 21, wherein the *Lactobacillus helveticus* satisfies at least one requirement selected from the group consisting of the following (1) to (3):
   (1) when the *Lactobacillus helveticus* is added at 1 × 10⁷ cells/well to a well seeded with 250 µL of human-derived peripheral-blood mononuclear cell fluid at a concentration of 8 × 10⁵ cells/mL, 100 ng/mL or more of IgA is produced;
   (2) when the *Lactobacillus helveticus* is added at 4 × 10⁶ cells/well to a well seeded with 200 µL of human-derived dendritic cell-like cells at a concentration of 1.25 × 10⁵ cells/mL, CD86 expression intensity becomes at least 1.5 times higher than that of a control without any lactic acid bacteria; and
   (3) when the *Lactobacillus helveticus* is added at 4 × 10⁶ cells/well to a well seeded with 200 µL of human-derived dendritic cell-like cells at a concentration of 1.25 × 10⁵ cells/mL, 300 pg/mL or more of IL-12 is produced.
Item 23.
   The composition according to Item 22, wherein the *Lactobacillus helveticus* satisfies the three requirements (1) to (3).
Item 24.
   The composition according to any one of Items 20 to 23, wherein at least one immunostimulatory function among the three immunostimulatory functions, the dendritic cell-like cell activation, the IgA production induction, and the IL-12 production induction possessed by the *Lactobacillus helveticus,* is higher than that of *Lactococcus lactis* JCM 5805 strain.
Item 25.
   The composition according to any one of Items 20 to 24, wherein the three immunostimulatory functions, the dendritic cell-like cell activation, the IgA production induction, and the IL-12 production induction, possessed by the *Lactobacillus helveticus,* are higher than those of *Lactococcus lactis* JCM 5805 strain.
Item 26.
   The composition according to any one of Items 20 to 25, wherein the immune stimulation is maintenance of immune function in a healthy individual.
Item 27.
   A lactic acid bacterium that is *Lactobacillus helveticus,* wherein the lactic acid bacterium has three functions: dendritic cell-like cell activation, IgA production induction, and IL-12 production induction.
Item 28.
   The lactic acid bacterium according to Item 27, wherein the lactic acid bacterium is *Lactobacillus helveticus* GCL1815 or *Lactobacillus helveticus* GCJ5-4B.
Item 29.
   The lactic acid bacterium according to Item 27 or 28, wherein the *Lactobacillus helveticus* satisfies at least one requirement selected from the group consisting of the following (1) to (3):
   (1) when the *Lactobacillus helveticus* is added at 1 × 10⁷ cells/well to a well seeded with 250 µL of human-derived peripheral-blood mononuclear cell fluid at a concentration of 8 × 10⁵ cells/mL, 100 ng/mL or more of IgA is produced;
   (2) when the *Lactobacillus helveticus* is added at 4 × 10⁶ cells/well to a well seeded with 200 µL of human-derived dendritic cell-like cells at a concentration of 1.25 × 10⁵ cells/mL, CD86 expression intensity becomes at least 1.5 times higher than that of a control without any lactic acid bacteria; and
   (3) when the *Lactobacillus helveticus* is added at 4 × 10⁶ cells/well to a well seeded with 200 µL of human-derived dendritic cell-like cells at a concentration of 1.25 × 10⁵ cells/mL, 300 pg/mL or more of IL-12 is produced.
Item 30.
   The lactic acid bacterium according to Item 29, wherein the *Lactobacillus helveticus* satisfies the three requirements (1) to (3).
Item 31.
   The lactic acid bacterium for use according to any one of Items 13 to 16, wherein at least one immunostimulatory function among the three immunostimulatory functions, the dendritic cell-like cell activation, the IgA production induction, and the IL-12 production induction possessed by the *Lactobacillus helveticus,* is higher than that of *Lactococcus lactis* JCM 5805 strain.
Item 32.
   The lactic acid bacterium according to any one of Items 27 to 31, wherein the three immunostimulatory functions, the dendritic cell-like cell activation, the IgA production induction, and the IL-12 production induction, possessed by the *Lactobacillus helveticus,* are higher than those of *Lactococcus lactis* JCM 5805 strain.
Item 33.
   An oral composition for maintaining immune function in a healthy individual, comprising *Lactobacillus helveticus* as an active ingredient, wherein the *Lactobacillus helveticus* has three functions: dendritic cell-like cell activation, IgA production induction, and IL-12 production induction.
Item 34.
   Use of *Lactobacillus helveticus* in the production of an agent for immune stimulation, wherein the *Lactobacillus helveticus* has three functions: dendritic cell-like cell activation, IgA production induction, and IL-12 production induction.
Item 35.
   A method for immune stimulation, comprising administering an effective amount of *Lactobacillus helveticus* to a subject in need thereof, wherein the *Lactobacillus helveticus* has three functions: dendritic cell-like cell activation, IgA production induction, and IL-12 production induction.
Item 36.
   A method for preventing or treating viral infection, comprising administering an effective amount of *Lactobacillus helveticus* to a subject in need thereof, wherein the *Lactobacillus helveticus* has three functions: dendritic cell-like cell activation, IgA production induction, and IL-12 production induction.
Item 37.
   The use according to Item 34 or the method according to Item 35 or 36, wherein the *Lactobacillus helveticus* is *Lactobacillus helveticus* GCL1815 or *Lactobacillus helveticus* GCJ5-4B.
Item 38.
   The use according to Item 34, the method according to Item 35 or 36, or the use or method according to Item 37, wherein the *Lactobacillus helveticus* satisfies at least one requirement selected from the group consisting of the following (1) to (3):
   (1) when the *Lactobacillus helveticus* is added at 1 × 10⁷ cells/well to a well seeded with 250 µL of human-derived peripheral-blood mononuclear cell fluid at a concentration of 8 × 10⁵ cells/mL, 100 ng/mL or more of IgA is produced;
   (2) when the *Lactobacillus helveticus* is added at 4 × 10⁶ cells/well to a well seeded with 200 µL of human-derived dendritic cell-like cells at a concentration of 1.25 × 10⁵ cells/mL, CD86 expression intensity becomes at least 1.5 times higher than that of a control without any lactic acid bacteria; and
   (3) when the *Lactobacillus helveticus* is added at 4 × 10⁶ cells/well to a well seeded with 200 µL of human-derived dendritic cell-like cells at a concentration of 1.25 × 10⁵ cells/mL, 300 pg/mL or more of IL-12 is produced.
Item 39.
   The use or method according to Item 38, wherein the *Lactobacillus helveticus* satisfies the three requirements (1) to (3).
Item 40.
   The use according to Item 34, the method according to Item 35 or 36, or the use or method according to any one of Items 37 to 39, wherein at least one immunostimulatory function among the three immunostimulatory functions, the dendritic cell-like cell activation, the IgA production induction, and the IL-12 production induction possessed by the *Lactobacillus helveticus,* is higher than that of *Lactococcus lactis* JCM 5805 strain.
Item 41.
   The use according to Item 34, the method according to Item 35 or 36, or the use or method according to any one of Items 37 to 40, wherein the three immunostimulatory functions, the dendritic cell-like cell activation, the IgA production induction, and the IL-12 production induction, possessed by the *Lactobacillus helveticus,* are higher than those of *Lactococcus lactis* JCM 5805 strain.
Item 42.
   The use according to Item 34, the method according to Item 35, or the use or method according to any one of Items 37 to 41, wherein the immune stimulation is maintenance of immune function in a healthy individual.
Item 43.
   The method according to any one of Items 35 to 42, wherein the *Lactobacillus helveticus* is administered orally.
Item 44.
   The use according to any one of Items 34 to 42, wherein the agent for immune stimulation is an oral composition.
Item 45.
   The method according to any one of Items 35 to 43, wherein the *Lactobacillus helveticus* is administered as a food or drink.
Item 46.
   The use according to any one of Items 34 to 42 and 44, wherein the agent for immune stimulation is a composition for food or drink.
Item 47.
   The use according to any one of Items 34 to 42, 44, and 46, wherein the agent for immune stimulation is a prophylactic or therapeutic agent for viral infection.

### Advantageous Effects of Invention

The present invention provides a novel agent for immune stimulation containing lactic acid bacteria of the genus *Lactobacillus* with high immunostimulatory activity.

### Brief Description of Drawings

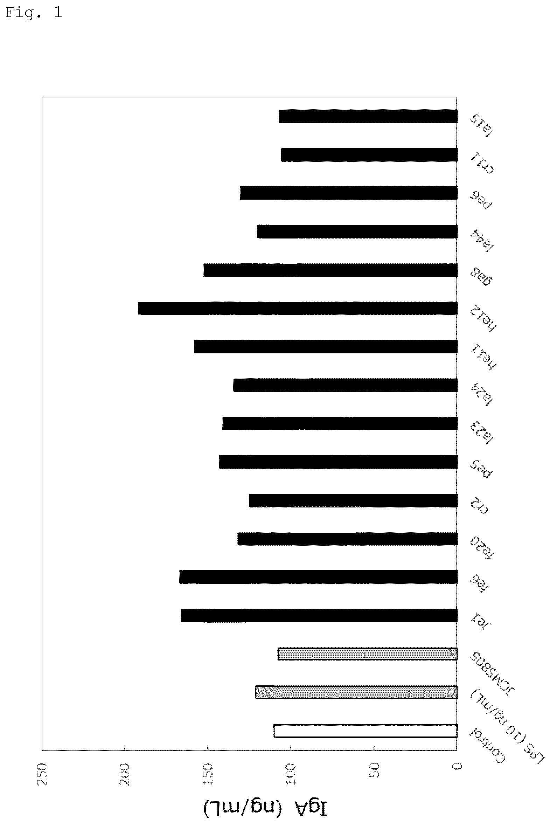

Fig. 1 shows the results of evaluating IgA production ability in Preparation Examples 1 and 2. The abbreviations in the figure are as follows: je1: *Lactobacillus jensenii 1,* fe6: *Limosilactobacillus fermentum* 6, fe20: *Limosilactobacillus fermentum* 20, cr2: *Lactobacillus crispatus* 2, pe5: *Lactiplantibacillus pentosus* 5, la23: *Lactococcus lactis 23,* la24: *Lactococcus lactis* 24, hell: *Lactobacillus helveticus* GCJ5-4B (accession number: NITE BP-03805), he12: *Lactobacillus helveticus* GCL1815 (accession number: NITE BP-03804), ga8: *Lactobacillus gasseri* 8, la44: *Lactococcus lactis* 44, pe6: *Lactiplantibacillus pentosus* 6, cr11: *Lactobacillus crispatus 11,* la15: *Lactococcus lactis 15*
Fig. 2 shows the results of evaluating dendritic cell-like cell activation in Preparation Examples 1 and 2.
Fig. 3 shows the results of evaluating NK cell activation in Preparation Examples 1 and 2.

### Description of Embodiments

### Agent for Immune Stimulation

The present invention provides an agent for immune stimulation containing *Lactobacillus helveticus* as an active ingredient, wherein the *Lactobacillus helveticus* has three functions: dendritic cell-like cell activation, IgA production induction, and IL-12 production induction.

Examples of *Lactobacillus helveticus* include *Lactobacillus helveticus* GCL1815 (including those described as *"Lactobacillus helveticus* GCD8-9D"; hereinafter, the same applies), *Lactobacillus helveticus* GCJ5-4B, etc., with *Lactobacillus helveticus* GCL1815 and *Lactobacillus helveticus* GCJ5-4B being preferred. In a preferred embodiment, examples of *Lactobacillus helveticus* include strains deposited on January 20, 2023, at the NITE Patent Microorganisms Depository of the National Institute of Technology and Evaluation (Room 122, 2-5-8 Kazusakamatari, Kisarazu-shi, Chiba Prefecture, Japan 292-0818) with accession numbers NITE BP-03804 and NITE BP-03805.

In the present invention, *Lactobacillus helveticus,* which is the active ingredient of the agent for immune stimulation, has all of the following three functions: dendritic cell-like cell activation, IgA production induction, and IL-12 production induction.

Specifically, first, *Lactobacillus helveticus,* which is the active ingredient of the agent for immune stimulation, has dendritic cell-like cell activation. Dendritic cells play a significant role in immune function. More specifically, for example, dendritic cells play an important role in innate immunity along with macrophages and NK cells. When foreign substances such as bacteria or viruses enter the body, dendritic cells first work to eliminate them. Furthermore, dendritic cells play an important role in acquired immunity by presenting antigens to T cells and B cells to transmit information about invading foreign substances, promoting the production of antibodies that specifically act against these foreign substances, and activating cytotoxic T cells.

Thus, dendritic cells are involved in both innate and acquired immunity, and have a significant impact on the overall immune system. There are various types of dendritic cells, and they can be broadly classified into plasmacytoid dendritic cells (pDC) and conventional dendritic cells (cDC). pDCs are the main producers of type I interferons, which show growth-inhibiting activity on viruses and contribute to NK cell activation, but have a weak antigen-presenting ability. On the other hand, cDCs not only produce IL-12, which activates NK cells, but also play an important role in inducing acquired immunity due to their strong antigen-presenting ability. IgA, a type of acquired immunity, is believed to contribute to the prevention of a wide range of infectious diseases because, unlike other immunoglobulins, it has low specificity and acts on many types of bacteria and viruses. cDCs, which activate both innate and acquired immunity, are one type of the cells that play key roles in the prevention of infectious diseases. In the present invention, the phrase "dendritic cell-like cell" is a collective term for cells including dendritic cells collected from living organisms as well as cells that have the functionality of cDCs, and that are derived from monocytes or stem cells, such as iPS cells and ES cells. The effect of lactic acid bacteria on dendritic cells in vivo can be evaluated by measuring the degree of dendritic cell-like cell activation.

Specifically, the dendritic cell-like cell activation by lactic acid bacteria can be evaluated by adding 4 × 10⁶ cells/well of lactic acid bacteria to wells each seeded with 200 µL of human-derived dendritic cell-like cells at a concentration of 1.25 × 10⁵ cells/mL, and measuring the expression intensity of CD86. More specifically, the measurement above can be performed according to the method described in the Examples of the present specification. In the present invention, among *Lactobacillus helveticus* strains, preferred strains are those that have a CD86 expression intensity measured according to the method above of, for example, 105% or more, preferably 130% or more, more preferably 145% or more, even more preferably 150% or more, still more preferably 180% or more, and particularly preferably 200% or more, as compared to a control without any lactic acid bacteria (based on the ratio represented by [CD86 expression intensity in a group with lactic acid bacteria (active ingredient of the present invention) added]/[CD86 expression intensity in control]). While the dendritic cell-like cells used for the measurement above are not limited, immortalized dendritic cells differentiated from immature dendritic cells (Mylc cells) as used in the Examples of the present specification can be typically used.

The IgA production induction by lactic acid bacteria can be evaluated by adding 1×10⁷ cells/well of the lactic acid bacteria to wells each seeded with 250 µL of human-derived peripheral-blood mononuclear cell fluid at a concentration of 8×10⁵ cells/mL and measuring produced IgA. More specifically, the measurement can be performed according to the method described in the Examples of the present application. In the present invention, among *Lactobacillus helveticus* strains, those that produce IgA at 100 ng/mL or more, more preferably 150 ng/mL or more, as measured according to the method above, are preferred. In the present invention, among *Lactobacillus helveticus* strains, preferred strains are those that produce IgA in an amount of, for example, 105% or more, preferably 110% or more, preferably 120% or more, preferably 140% or more, and preferably 170% or more, measured according to the method above, as compared to a control without any lactic acid bacteria (based on the ratio represented by [IgA production amount (ng/mL) in a group with lactic acid bacteria (active ingredient of the present invention) added]/[IgA production amount (ng/mL) in control]).

The IL-12 production induction by lactic acid bacteria can be evaluated by, for example, adding 4 × 10⁶ cells/well of the lactic acid bacteria to wells each seeded with 200 µL of human-derived dendritic cell-like cells at a concentration of 1.25 × 10⁵ cells/mL and measuring the produced IL-12. More specifically, the measurement can be performed according to the method described in the Examples of the present specification. In the present invention, among *Lactobacillus helveticus* strains, preferable strains are those that preferably produce 300 pg/mL or more, more preferably 600 pg/mL or more, and even more preferably 900 pg/mL or more of IL-12 as measured according to the method above.

In the present invention, among *Lactobacillus helveticus* strains, preferable strains are those that produce IL-12 in an amount of preferably 110% or more, more preferably 180% or more, even more preferably 290% or more, still more preferably 500% or more, and particularly preferably 1000% or more measured according to the method above, as compared to a control without any lactic acid bacteria (based on the ratio represented by [IL-12 production (pg/mL) in a group with lactic acid bacteria (active ingredient of the present invention) added]/[IL-12 production (pg/mL) in control]).

In a more preferred embodiment, it is preferable to use *Lactobacillus helveticus* that exhibits at least one (preferably at least two, more preferably all three) immunostimulatory function selected from the group consisting of dendritic cell-like cell activation, IgA production induction, and IL-12 production induction, higher than that of *Lactococcus lactis* JCM 5805 strain. The measurement methods for dendritic cell-like cell activation, IgA production induction, and IL-12 production induction in this embodiment are the same as those described above. The functions of the lactic acid bacteria JCM 5805 strain can be measured by performing the same operations as described above, except for using the lactic acid bacteria JCM 5805 strain instead of *Lactobacillus helveticus,* which is the active ingredient of the agent for immune stimulation of the present invention.

In the present invention, the *Lactobacillus helveticus* for use can be dead bacteria (such as heat-killed bacteria) or live bacteria. In the present invention, the *Lactobacillus helveticus* can be used singly or in a combination of two or more strains.

In the present invention, the lactic acid bacteria described above are used as an active ingredient in an agent for immune stimulation. The use of "immune stimulation" can be for the aforementioned dendritic cell-like cell activation, IgA production induction, IL-12 production induction, and others. In the present invention, the phrase "immune stimulation" includes not only improving the immune function of humans with weakened immune function, but also maintaining the immune function of healthy individuals.

In the present invention, the active ingredient of the present invention, *Lactobacillus helveticus* itself, may be used as an agent for immune stimulation, or *Lactobacillus helveticus* may be used in the form of a composition containing powder of the lactic acid bacteria in combination with various carriers that are pharmaceutically acceptable or that can be added to food (e.g., isotonic agents, chelating agents, stabilizers, pH adjusters, preservatives, antioxidants, solubilizing agents, thickeners, excipients, and binders). In this embodiment, the content of *Lactobacillus helveticus* in the composition of the agent for immune stimulation is not limited, and can be appropriately set within the range of, for example, 50 mass% or more, 60 mass% or more, 70 mass% or more, 80 mass% or more, 90 mass% or more, 95 mass% or more, 99 mass% or more, etc. The number of *Lactobacillus helveticus* in the powder of *Lactobacillus helveticus* used for the agent for immune stimulation is not limited, and can be appropriately set within the range of, for example, 100 million to 100 trillion/g, preferably 10 billion to 10 trillion/g, more preferably 50 billion to 1 trillion/g, even more preferably 100 billion/g or more, and particularly preferably 300 billion/g or more.

Examples of isotonic agents include sugars, such as glucose, trehalose, lactose, fructose, mannitol, xylitol, and sorbitol; polyhydric alcohols, such as glycerin, polyethylene glycol, and propylene glycol; and inorganic salts, such as sodium chloride, potassium chloride, and calcium chloride. These isotonic agents can be used singly or in a combination of two or more.

Examples of chelating agents include edetates, such as disodium edetate, calcium disodium edetate, trisodium edetate, tetrasodium edetate, and calcium edetate, as well as ethylenediaminetetraacetate, nitrilotriacetic acid or its salt, sodium hexametaphosphate, and citric acid. These chelating agents can be used singly or in a combination of two or more.

Examples of stabilizers include sodium bisulfite.

Examples of pH adjusters include acids, such as hydrochloric acid, carbonic acid, acetic acid, and citric acid, alkali metal hydroxides, such as sodium hydroxide and potassium hydroxide, alkali metal carbonates or hydrogencarbonate, such as sodium carbonate, alkali metal acetates, such as sodium acetate, alkali metal citrates, such as sodium citrate, and bases, such as trometamol. These pH adjusters can be used singly or in a combination of two or more.

Examples of preservatives include sorbic acid, potassium sorbate, p-hydroxybenzoates, such as methyl p-hydroxybenzoate, ethyl p-hydroxybenzoate, propyl p-hydroxybenzoate, and butyl p-hydroxybenzoate, quaternary ammonium salts, such as chlorhexidine gluconate, benzalkonium chloride, benzethonium chloride, and cetylpyridinium chloride, alkyl polyaminoethyl glycine, chlorobutanol, polyquaternium, polyhexamethylene biguanide, and chlorhexidine. These preservatives can be used singly or in a combination of two or more.

Examples of antioxidants include sodium bisulfite, dried sodium sulfite, sodium pyrosulfite, and mixed tocopherol concentrate. These antioxidants can be used singly or in a combination of two or more.

Examples of solubilizing agents include sodium benzoate, glycerin, D-sorbitol, glucose, propylene glycol, hydroxypropyl methylcellulose, polyvinylpyrrolidone, macrogol, and D-mannitol. These solubilizing agents can be used singly or in a combination of two or more.

Examples of thickeners include polyethylene glycol, methyl cellulose, ethyl cellulose, carmellose sodium, xanthan gum, sodium chondroitin sulfate, hydroxyethyl cellulose, hydroxypropyl cellulose, hydroxypropyl methylcellulose, polyvinylpyrrolidone, and polyvinyl alcohol. These thickeners can be used singly or in a combination of two or more.

Examples of excipients include lactose, corn starch, L-cysteine, trehalose, maltitol, and sorbitol. These excipients can be used singly or in a combination of two or more.

Examples of binders include crystalline cellulose, starch, sucrose, hydroxypropyl cellulose, gelatin, powdered gum arabic, polyvinylpyrrolidone, pullulan, dextrin, cyclodextrin, methyl cellulose, ethyl cellulose, hydroxymethyl cellulose, hydroxypropyl cellulose, hydroxypropyl methyl cellulose, polyvinyl alcohol, and polyethylene glycol. These binders can be used singly or in a combination of two or more.

The composition described above may further contain substances known to have immune-stimulating functions in addition to the *Lactobacillus helveticus* described above. Examples of substances known to have immune-stimulating functions include vitamin C, vitamin A, and zinc. These substances can be used singly or in a combination of two or more.

By consuming the agent for immune stimulation of the present invention, the subject (preferably a mammal, such as a human) experiences an immunostimulatory effect. The intake amount of the agent for immune stimulation of the present invention is not limited, and the daily intake of *Lactobacillus helveticus,* which is the active ingredient, can be appropriately set within the range of, for example, 10 million to 10 trillion cells, preferably 100 million to 1 trillion cells, more preferably 1 billion to 100 billion cells, even more preferably 5 billion to 30 billion cells, and particularly preferably 10 billion to 20 billion cells. While the intake amount of the agent for immune stimulation of the present invention based on the weight of *Lactobacillus helveticus* is also not limited, the daily intake of *Lactobacillus helveticus* as the active ingredient can be appropriately set, for example, within the range of 1 mg to 10 g, preferably 10 mg to 1000 mg, more preferably 40 mg to 300 mg, and even more preferably 200 mg or more.

### Oral Composition

In another embodiment, the present invention provides an oral composition containing the agent for immune stimulation. Thus, the present invention provides an oral composition containing the *Lactobacillus helveticus.* The oral composition includes food or drink compositions and pharmaceutical compositions. In the present invention, the food or drink compositions include health-functional foods (foods with nutrient function claims, foods for specified health uses, and foods with function claims).

Examples of food or drink compositions include beverages, such as vegetable juice beverages, fruit juice beverages, mixed vegetable and fruit juice beverages, fermented milk beverages, and almond-containing beverages; and foods, such as ice cream, sherbets, almond-containing foods, biscuits (e.g., cream sandwich biscuits), chocolate (including quasi-chocolate), and fermented milk products (yogurt and cheese). The ice cream is preferably lacto-ice cream (such as lacto-ice cream containing fermented milk). The cream sandwich biscuits are preferably those with the *Lactobacillus helveticus* added to the cream. The quasi-chocolate includes corn-containing quasi-chocolate. In the present invention, among these foods and drinks, dairy products and foods or drinks containing dairy products are preferred. The dairy products include fermented milk, ice cream, and milk beverages. Examples of foods or drinks containing dairy products include cream sandwich biscuits and milk chocolate. Preferable sherbets include milk-containing sherbets (including sherbets containing fermented milk, and like desserts). The food or drink composition of the present invention also includes supplements. The supplements are preferably sports supplements (e.g., amino acid-containing sports supplements). Examples of amino acid-containing sports supplements include glutamine-containing sports supplements.

The details of the active ingredient *Lactobacillus helveticus* in the embodiment of the oral composition, the intake amount, and other details are as described in the explanation of the agent for immune stimulation above. In this embodiment, the content of *Lactobacillus helveticus* in the oral composition is not particularly limited, and can be appropriately set within the range of, for example, 0.00001 to 99 mass%, preferably 0.0001 to 50 mass%, more preferably 0.001 to 10 mass%, even more preferably 0.01 to 1 mass%, and particularly preferably 0.1 mass% or more. The oral composition of the present invention can be used for immune-stimulating purposes.

### Prophylactic or Therapeutic Agent for Viral Infection

The administration of specific *Lactobacillus helveticus,* which is the active ingredient of present invention, can provide an immune-stimulating effect. Thus, the immune-stimulating effect provided by the present invention is expected to provide a prophylactic or therapeutic effect on viral infections. Thus, the present invention provides a prophylactic or therapeutic agent for viral infection containing the agent for immune stimulation described above. The present invention also provides a prophylactic or therapeutic agent for viral infection containing the *Lactobacillus helveticus* described above. Examples of target viruses include, but are not limited to, influenza virus, coronavirus, adenovirus, RS virus, human metapneumovirus, and rhinovirus. The details of *Lactobacillus helveticus* as the active ingredient in the embodiment of the prophylactic or therapeutic agent for viral infection, the intake amount, and other details are as described in the explanation of the agent for immune stimulation and the oral composition.

### Lactobacillus helveticus

The present invention can provide immune-stimulating effects and prophylactic or therapeutic effects for viral infections due to the *Lactobacillus helveticus* described above. The *Lactobacillus helveticus* described above is novel. Thus, the present invention also provides the *Lactobacillus helveticus* itself. The method of using the *Lactobacillus helveticus* in this embodiment and other details are as described in the explanation of the agent for immune stimulation, the oral composition, and the prophylactic or therapeutic agent for viral infection.

### Examples

### Preparation of Killed Lactic Acid Bacteria Powder

### Preparation Example 1

An in-house lactic acid bacteria strain was statically cultured in MRS medium (Merck Millipore) at 30°C or 37°C for 48 hours. After culture, the bacteria were collected by centrifugation at 8,000×G for 10 minutes, washed three times with endotoxin-free physiological saline (simply "physiological saline" below), and then sterilized by autoclaving at 121°C for 15 minutes. Subsequently, the bacterial cells were freeze-dried and adjusted with physiological saline so as to give a concentration of 10 mg/mL, thereby obtaining a suspension of the lactic acid bacteria.

### Preparation Example 2

Heat-killed cells of *Lactococcus lactis* JCM5805 strain ("JCM5805 strain" below) were prepared in the same manner as in Preparation Example 1. Subsequently, the heat-killed bacteria cells were freeze-dried and adjusted with physiological saline so as to give a concentration of 10 mg/mL, thereby obtaining a suspension of the lactic acid bacteria. The strains with "JCM" in their name indicate bacterial strains distributed by RIKEN (RIKEN, The Institute of Physical and Chemical Research).

### Screening Based on IgA Production-inducing Ability

### Preparation of Peripheral Blood Mononuclear Cell Culture Medium ("PBMC Medium" below)

PBMC medium was prepared by adding the following to RPMI 1640 (Thermo Fisher Scientific) to achieve the indicated percentages: 10 mass% of heat-inactivated fetal bovine serum (FBS) (Thermo Fisher Scientific), 1 mass% of an MEM vitamin solution (Thermo Fisher Scientific), 1 mass% of an MEM non-essential amino acid solution (Thermo Fisher Scientific), 1 mass% of a penicillin-streptomycin solution (Fujifilm Wako Pure Chemical Corporation), 1 mass% of a sodium pyruvate solution (Thermo Fisher Scientific), and 0.1 mass% of 2-mercaptoethanol (Thermo Fisher Scientific).

### Preparation of Human Peripheral Blood Mononuclear Cells (PBMCs)

Frozen human peripheral blood mononuclear cells (PBMCs) derived from a healthy donor (Astarte Biologics) were quickly thawed in a 37°C water bath, added to a 50 mL centrifuge tube containing 10 mL of RPMI1640 medium supplemented with 10% FBS, and then gently mixed by inversion. The PBMCs were precipitated by centrifugation at 200×G for 5 minutes, and the supernatant was removed with an aspirator, followed by adding 10 mL of PBMC medium to the pelleted PBMCs to resuspend the PBMCs, thereby obtaining a suspension of PBMCs.

### Seeding of PBMCs, Addition of Lactic Acid Bacteria, and Culture

20 µL of the suspension of PBMCs and 2 µL of acridine orange (Logos Biosystems) were added to a 1.5 mL microcentrifuge tube and mixed well. Subsequently, the cell concentration was calculated using a cell counter (Logos Biosystems, L20001). The suspension of PBMCs was diluted with PBMC medium to a cell concentration of 8 × 10⁵ cells/mL, and 250 µL of the suspension of PBMCs was seeded into each well of a 96-well plate (TPP). Furthermore, the suspension of lactic acid bacteria from Preparation Example 1 was added to each well of the 96-well plate so as to achieve 50 µg/mL. As a control, wells without any lactic acid bacteria were also prepared. The PBMCs were then cultured statically for 120 hours in a CO₂ incubator at 37°C with 5% CO₂.

### IgA Measurement

The 96-well plate, containing PBMCs cultured for 120 hours, was taken out, and 250 µL of the culture fluid was taken and placed in 8-strip microtubes. The PBMCs were precipitated by centrifugation at 200 × G for 10 minutes, and 220 µL of the culture supernatant was placed in another 8-strip microtubes, followed by further centrifugation at 1,500 × G for 10 minutes. The resulting supernatant was used for IgA quantification by ELISA. For IgA quantification, a Human IgA ELISA Kit (Abcam) was used, and the IgA concentration was measured according to the Protocol Booklet included in the kit. To ensure reproducibility of the experiment, the procedure from PBMC seeding to IgA measurement was performed twice.

### Test Results

Of the lactic acid bacteria in the culture fluid in which PBMCs were cultured with the lactic acid bacteria of Preparation Example 1, lactic acid bacteria that resulted in an IgA concentration at least twice that of the culture fluid (control) in which PBMCs were cultured without adding lactic acid bacteria were selected. As a result of screening under the conditions above, 70 strains of lactic acid bacteria were selected as lactic acid bacteria with an IgA production-inducing ability. Table 1 shows a list of those lactic acid bacteria. The IgA production rate was calculated using the following formula: IgA production rate = IgA concentration in PBMC culture fluid with lactic acid bacteria/IgA concentration in PBMC culture fluid without lactic acid bacteria

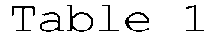

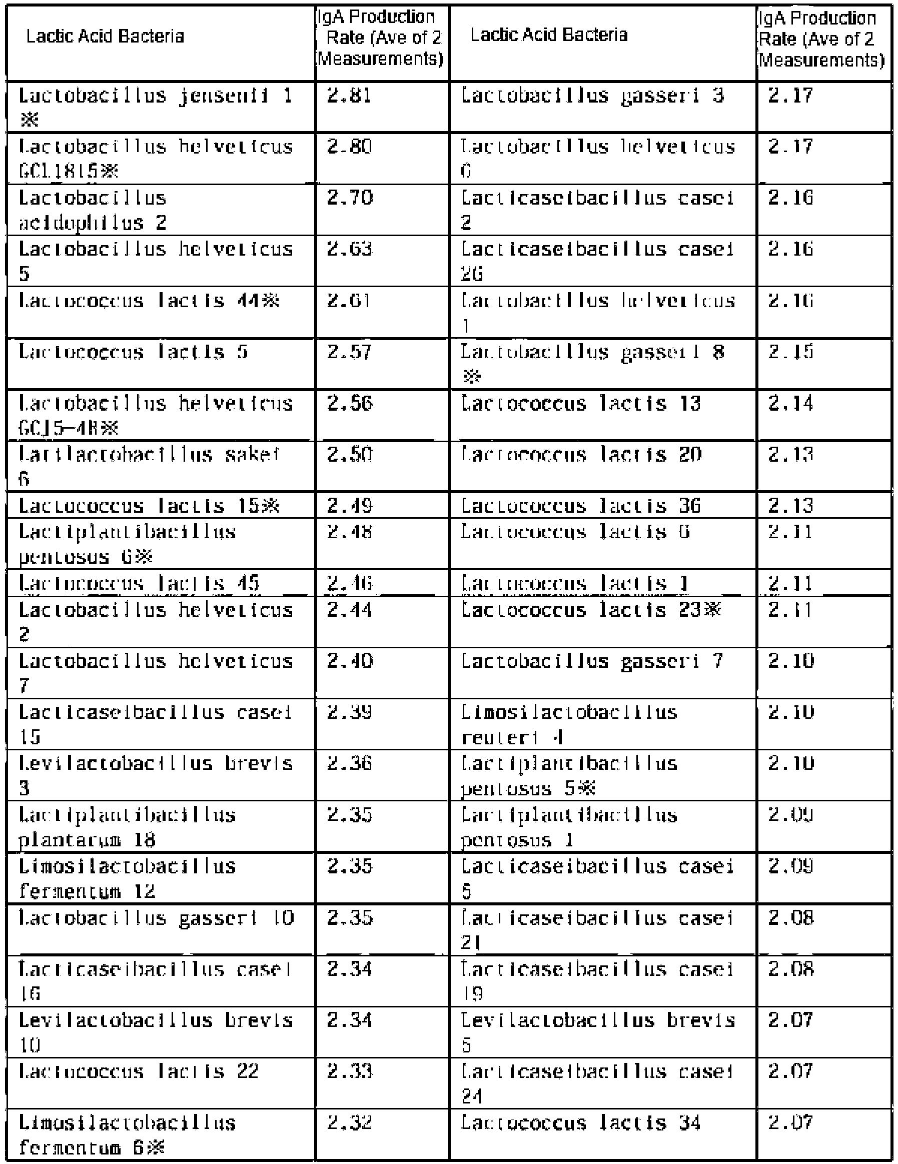

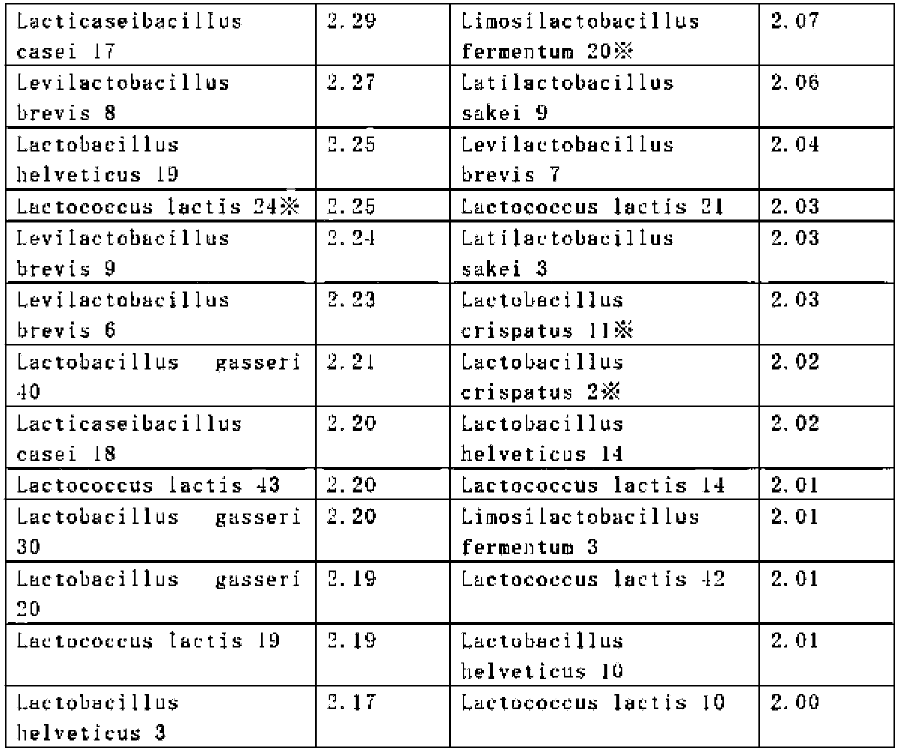

From these lactic acid bacteria, 14 strains of lactic acid bacteria were selected for subsequent tests from the viewpoint of different bacterial types, ease of culture, and other factors. The selected lactic acid bacteria are marked with an asterisk ( 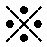 ) in Table 1.

### Evaluation of IgA Production Induction

### Isolation of Peripheral Blood Mononuclear Cells from Human Peripheral Blood

Blood samples of 20 mL each were collected from 12 healthy adult volunteers and placed in blood collection tubes containing an anticoagulant (sodium heparin). First, the Lymphoprep^{™} Tube (AXS) was centrifuged at 400 × G for 1 minute to settle the Lymphoprep^{™} to the bottom of the Lymphoprep^{™} Tube before use. Second, after the blood and physiological saline were mixed in a ratio of 1:1 in a 50 mL centrifuge tube, 20 to 30 mL of the mixture was transferred to the Lymphoprep^{™} Tube and centrifuged at 800 × G for 20 minutes at room temperature. From the layers formed by centrifugation, the layer of a solution containing peripheral blood mononuclear cells was collected and placed in another 50 mL centrifuge tube by using a Pasteur pipette. 20 mL of physiological saline was added to the 50 mL centrifuge tube containing the collected peripheral blood mononuclear cells, and the cells were pelleted by centrifugation at 250 × G for 10 minutes. The supernatant was removed using an aspirator, and 1 mL of PBMC medium was added to the pelleted peripheral blood mononuclear cells to suspend the cells, thereby obtaining a peripheral blood mononuclear cell fluid.

### Seeding of Peripheral Blood Mononuclear Cells, Addition of Lactic Acid Bacteria, and Culture

90 µL of PBMC medium and 10 µL of the peripheral blood mononuclear cell fluid were measured and placed in a 1.5 mL microcentrifuge tube and mixed well. Then, 20 µL of this mixture was transferred to another 1.5 mL microcentrifuge tube, and 2 µL of acridine orange (Logos Biosystems) was added and mixed thoroughly. Subsequently, the cell concentration was calculated using a cell counter (Logos Biosystems, L20001). The peripheral blood mononuclear cell fluid was diluted with PBMC medium to a concentration of 8 × 10⁵ cells/mL, and 250 µL of the diluted fluid was seeded into each well of a 96-well plate (TPP). Additionally, the suspension of lactic acid bacteria from Preparation Example 1 and the suspension of lactic acid bacteria from Preparation Example 2 were each added at 1 × 10⁷ cells/well to the wells of the 96-well plate, along with addition of lipopolysaccharide (Fujifilm Wako Pure Chemical Corporation) ("LPS" below) so as to give a final concentration of 10 ng/mL as a positive control. Additionally, wells without lactic acid bacteria were also prepared as a control. The cells were cultured statically in a CO₂ incubator at 37°C with 5% CO₂ for 120 hours.

### IgA Measurement

The 96-well plate, containing peripheral blood mononuclear cells cultured for 120 hour, was taken out, and 250 µL of the culture fluid was collected and placed in 8-strip microtubes. The peripheral blood mononuclear cells were precipitated by centrifugation at 200 × G for 10 minutes, and 220 µL of the culture supernatant was placed in another 8-strip microtubes, followed by further centrifugation at 1,500 × G for 10 minutes. The resulting supernatant was used for IgA quantification by ELISA. For IgA quantification, a Human IgA ELISA Kit (Abcam) was used, and the IgA concentration was measured according to the Protocol Booklet included in the kit.

### Evaluation of Dendritic Cell-like Cell Activation

### Preparation of Differentiation Medium

Mylc-specific medium B (MiCAN Technologies Inc.) and Mylc-specific medium supplement B (MiCAN Technologies Inc.) were mixed in a ratio of 200:1 to obtain a differentiation medium.

### Preparation of Suspension of Lactic Acid Bacteria (1 mg/mL)

The suspensions of lactic acid bacteria obtained in Preparation Examples 1 and 2 were diluted 10-fold with physiological saline to obtain 1 mg/mL suspensions of lactic acid bacteria. The 1 mg/mL suspensions of lactic acid bacteria were measured for bacterial concentration according to the Breed method and diluted with the differentiation medium to achieve a concentration of 4×10⁷ cells/mL.

### Seeding of Dendritic Cells, Addition of Lactic Acid Bacteria, and Culture

aMylc-2-A differentiated cells, which were immortalized dendritic cells ("dendritic cells" below) purchased from MiCAN Technologies Inc. and induced to differentiate for 3 days, were collected. Subsequently, the dendritic cells were precipitated by centrifugation at 300 × G for 5 minutes, and the supernatant was removed by using an aspirator, followed by adding 2 mL of the differentiation medium to the pelleted dendritic cells. After the cell concentration was calculated by using a cell counter, the dendritic cells were diluted with the differentiation medium to a concentration of 2.5 × 10⁵ cells/mL, followed by seeding 100 µL of the dendritic cells into each well of a Nunclon Sphera 96-well round-bottom plate (Thermo Fisher Scientific). Additionally, 100 µL of a suspension of lactic acid bacteria (4×10⁷ cells/mL) was added to each well, while 100 µL of LPS was added to the wells as a positive control so as to achieve a final concentration of 1 ng/mL. Wells without lactic acid bacteria were also prepared as a control. The cells were cultured statically in a CO₂ incubator at 37°C with 5% COₛ for 24 hours.

### Collection of Dendritic Cells and Culture Supernatant

The 96-well plate, containing dendritic cells cultured for 24 hours, was taken out, and 200 µL of the culture fluid was taken and placed in 8-strip microtubes. The dendritic cells were precipitated by centrifugation at 500 × G for 10 minutes, and 140 µL of the culture supernatant was placed in another 8-strip microtubes. The culture supernatant remaining in the 8-strip microtubes was removed by using an aspirator, thereby obtaining precipitates of dendritic cells.

### Preparation of Reagent for Measuring Cell Surface Marker (CD86)

### 1. FcR Blocking Solution

An eBioscience^{™} Flow Cytometry Staining Buffer (Invitrogen) ("staining buffer" below) and an Fc Receptor binding inhibitor (eBioscience, Inc.) were mixed in a ratio of 19:1 to obtain an FcR blocking solution.

### 2. Antibody Solution

The staining buffer and PE anti-human CD86 Antibody were mixed in a ratio of 49:1 to obtain an antibody solution.

### Measurement of Cell Surface Marker (CD86)

200 µL of the staining buffer was added to the dendritic cell precipitates to suspend them. The suspension was centrifuged at 500 × G for 5 minutes, and the supernatant was removed by using an aspirator. 20 µL of the FcR blocking solution was added to resuspend the cell precipitates, which were then allowed to stand on ice for 20 minutes. Furthermore, 20 µL of the antibody solution was added to resuspend the cells, and then the suspension was allowed to stand on ice for 30 minutes. After being left to stand, the cells were suspended by adding 160 µL of the staining buffer and centrifuged at 500 × G for 5 minutes, followed by removing the supernatant using an aspirator. Subsequently, 200 µL of the staining buffer was added to resuspend the cells, which were then centrifuged at 500 × G for 5 minutes, followed by removing the supernatant using an aspirator. The solution prepared by adding 200 µL of the staining buffer to resuspend the cells was measured for expression intensity of a cell surface marker (CD86) by using a flow cytometer (SA3800, Sony Corporation, laser wavelength: 488 nm).

### Evaluation of NK Cell Activation

IL-12 measurement was performed to evaluate NK cell activation by using the culture supernatant obtained according to the method described in the Collection of Dendritic Cells and Culture Supernatant section above. For IL-12 measurement, a Human IL-12 p70 ELISA Kit (Abcam) was used, and the IL-12 concentration was measured according to the Protocol Booklet included in the kit.

### Evaluation of IgA Production Induction

Fig. 1 shows the results of evaluating the IgA production ability of Preparation Example 1 and Preparation Example 2. As shown in Fig. 1, compared to the case without the addition of lactic acid bacteria (control), culturing with the lactic acid bacteria from Preparation Example 1 significantly increased the IgA production-inducing ability of peripheral blood mononuclear cells, except for cr2, la44, cr11, and la15. Additionally, as compared to the case with the JCM5805 strain of Preparation Example 2 added, culturing with lactic acid bacteria from Preparation Example 1 significantly increased the IgA production-inducing ability of peripheral blood mononuclear cells, except for cr2, la44, cr11, and la15. In animals, including humans, ingesting these types of lactic acid bacteria is expected to enhance IgA production, thereby preventing the entry of viruses and bacteria into the body and improving infection defense capabilities.

### Evaluation of Dendritic Cell-like Cell Activation

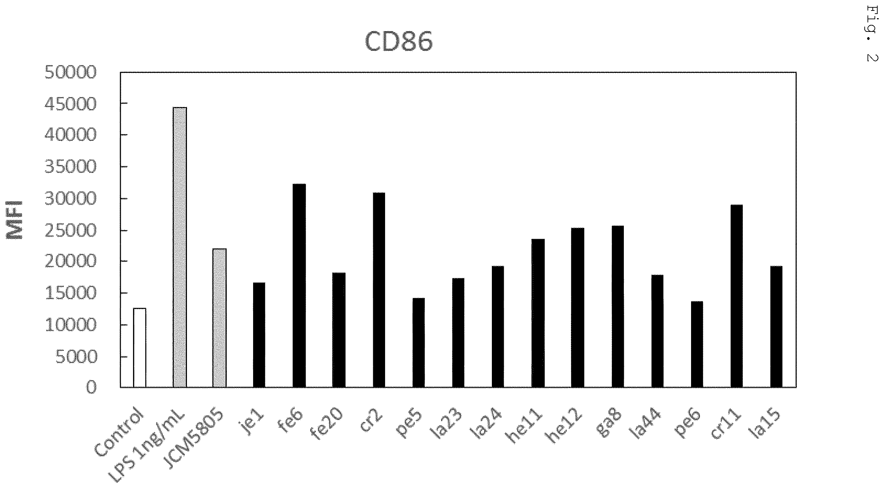

Fig. 2 shows the results of evaluating the dendritic cell-like cell activation of Preparation Example 1 and Preparation Example 2. The dendritic cell-like cell activation was evaluated by measuring the expression intensity of a cell surface marker (CD86). As shown in Fig. 2, compared to the case without the addition of lactic acid bacteria (control), culturing with the lactic acid bacteria from Preparation Example 1 significantly activated dendritic cells, except for pe6. Additionally, as compared to the case with the JCM5805 strain of Preparation Example 2 added, culturing with the lactic acid bacteria from Preparation Example 1 significantly activated dendritic cells, except for je1, fe20, pe5, la23, la24, la44, pe6, and la15. In animals, including humans, ingesting these types of lactic acid bacteria is expected to activate dendritic cells, thereby stimulating the overall immune system.

### Evaluation of NK Cell Activation

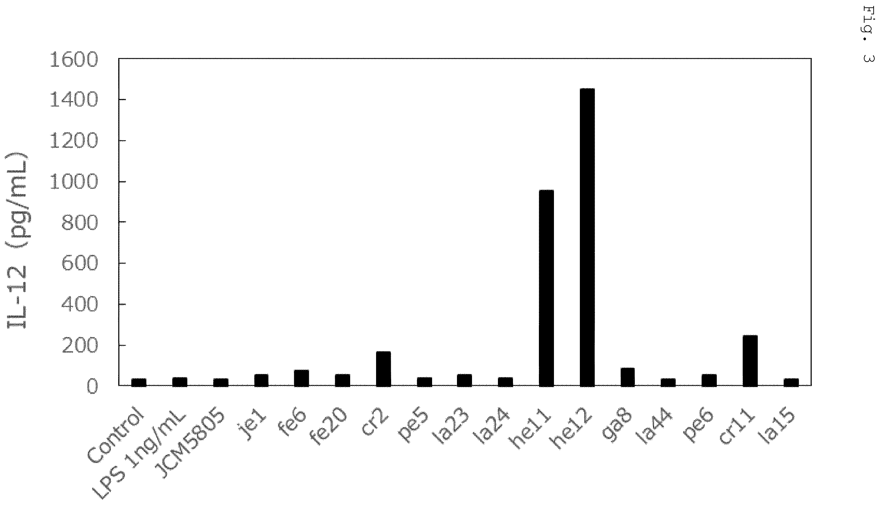

Fig. 3 shows the results of evaluating NK cell activation of Preparation Example 1 and Preparation Example 2. NK cell activation was evaluated by measuring IL-12 concentration. As shown in Fig. 3, compared to the case without the addition of lactic acid bacteria (control), culturing with the lactic acid bacteria from Preparation Example 1 significantly activated NK cells, except for la44. In particular, hell and he12 notably activated NK cells. Additionally, as compared to the case with the JCM5805 strain of Preparation Example 2 added, culturing with the lactic acid bacteria from Preparation Example 1 significantly activated NK cells. In animals, including humans, ingesting these types of lactic acid bacteria is expected to activate NK cells, thereby enhancing their action of attacking cells infected with bacteria or viruses, preventing the spread of infections and improving infection defense capabilities.

As shown in the tests above, hell and he12 demonstrated all three functions: dendritic cell-like cell activation, IgA production induction, and IL-12 production induction. Specifically, hell and he12 exhibited all of the following functions (1) to (3):
(1) when the *Lactobacillus helveticus* is added at 1 × 10⁷ cells/well to a well seeded with 250 µL of human-derived peripheral-blood mononuclear cell fluid at a concentration of 8 × 10⁵ cells/mL, 100 ng/mL or more of IgA is produced;
(2) when the *Lactobacillus helveticus* is added at 4 × 10⁶ cells/well to a well seeded with 200 µL of human-derived dendritic cell-like cells at a concentration of 1.25 × 10⁵ cells/mL, CD86 expression intensity becomes at least 1.5 times higher than that of a control without any lactic acid bacteria; and
(3) when the *Lactobacillus helveticus* is added at 4 × 10⁶ cells/well to a well seeded with 200 µL of human-derived dendritic cell-like cells at a concentration of 1.25 × 10⁵ cells/mL, 300 pg/mL or more of IL-12 is produced.

Additionally, hell and he12 showed that their three immunostimulatory functions, dendritic cell-like cell activation, IgA production induction, and IL-12 production induction, were all higher than those of the JCM 5805 strain.

### Formulation Example 1

Ice cream containing killed lactic acid bacteria powder was obtained from the ingredients shown in Table 2. Specifically, first, egg yolks and sugar were placed in a bowl and well mixed. In a separate pot, dairy cream, cow milk, and killed lactic acid bacteria powder were placed and heated. When bubbles started to form around the edge of the pot, the pot was removed from heat. This heated mixture was gradually added to the bowl containing beaten egg yolks and sugar with stirring. The obtained mixture was placed in a metal container to cool down, covered with a lid, and refrigerated at -20°C. After 3 hours, the entire mixture was stirred, and then stirred again 4 times at 30-minute intervals, thereby obtaining ice cream containing killed lactic acid bacteria powder.

**Table 2**

| | |
|---|---|
| Cow Milk | 57 g |
| Egg Yolk | 29 g |
| Sugar | 13 g |
| Killed Lactic Acid Bacteria Powder of Preparation Example 1 | 1 g |
| Total | 100 g |

### Formulation Example 2

Yogurt containing killed lactic acid bacteria powder was obtained from the ingredients shown in Table 3.

**Table 3**

| | |
|---|---|
| Yogurt (BifiX Yogurt, Non-sugar Plane Yogurt: Ezaki Glico Co., Ltd.) | 99 g |
| Killed Lactic Acid Bacteria Powder of Preparation Example 1 | 1g |
| Total | 100 g |

### Formulation Example 3

Chocolate containing killed lactic acid bacteria powder was obtained from the ingredients shown in Table 4. Specifically, chocolate was melted in a water bath at about 50°C, and then killed lactic acid bacteria powder was added and well mixed. The mixture was poured into a mold to shape, and left at room temperature to allow it to solidify, thereby obtaining chocolate containing killed lactic acid bacteria powder.

**Table 4**

| | |
|---|---|
| Chocolate | 99 g |
| Killed Lactic Acid Bacteria Powder of Preparation Example 1 | 1 g |
| Total | 100 g |

## Claims

1. An agent for immune stimulation, comprising *Lactobacillus helveticus* as an active ingredient, wherein the *Lactobacillus helveticus* has three functions: dendritic cell-like cell activation, IgA production induction, and IL-12 production induction.

2. The agent for immune stimulation according to claim 1, wherein the *Lactobacillus helveticus* is *Lactobacillus helveticus* GCL1815 or *Lactobacillus helveticus* GCJ5-4B.

3. The agent for immune stimulation according to claim 1 or 2, wherein the *Lactobacillus helveticus* satisfies at least one requirement selected from the group consisting of the following (1) to (3):
(1) when the *Lactobacillus helveticus* is added at 1 × 10⁷ cells/well to a well seeded with 250 µL of human-derived peripheral-blood mononuclear cell fluid at a concentration of 8 × 10⁵ cells/mL, 100 ng/mL or more of IgA is produced;
(2) when the *Lactobacillus helveticus* is added at 4 × 10⁶ cells/well to a well seeded with 200 µL of human-derived dendritic cell-like cells at a concentration of 1.25 × 10⁵ cells/mL, CD86 expression intensity becomes at least 1.5 times higher than that of a control without any lactic acid bacteria; and
(3) when the *Lactobacillus helveticus* is added at 4 × 10⁶ cells/well to a well seeded with 200 µL of human-derived dendritic cell-like cells at a concentration of 1.25 × 10⁵ cells/mL, 300 pg/mL or more of IL-12 is produced.

4. The agent for immune stimulation according to claim 3, wherein the *Lactobacillus helveticus* satisfies the three requirements (1) to (3).

5. The agent for immune stimulation according to claim 1 or 2, wherein at least one immunostimulatory function among the three immunostimulatory functions, the dendritic cell-like cell activation, the IgA production induction, and the IL-12 production induction possessed by the *Lactobacillus helveticus,* is higher than that of *Lactococcus lactis* JCM 5805 strain.

6. The agent for immune stimulation according to claim 1 or 2, wherein the three immunostimulatory functions, the dendritic cell-like cell activation, the IgA production induction, and the IL-12 production induction, possessed by the *Lactobacillus helveticus,* are higher than those of *Lactococcus lactis* JCM 5805 strain.

7. The agent for immune stimulation according to claim 1 or 2, wherein the immune stimulation is maintenance of immune function in a healthy individual.

8. An oral composition for immune stimulation, comprising the agent for immune stimulation of claim 1 or 2.

9. The oral composition according to claim 8, which is a composition for food or drink.

10. A prophylactic or therapeutic agent for viral infection, comprising the agent for immune stimulation of claim 1 or 2.

11. A lactic acid bacterium that belongs to the genus *Lactobacillus,* with accession number NITE BP-03804 at the NITE Patent Microorganism Depository (NPMD) of the National Institute of Technology and Evaluation (NITE).

12. A lactic acid bacterium that belongs to the genus *Lactobacillus,* with accession number NITE BP-03805 at the NITE Patent Microorganism Depository (NPMD) of the National Institute of Technology and Evaluation (NITE).
